# EUROPEAN PATENT SPECIFICATION

(11) **EP 1 372 700 B1**
(45) Date of publication and mention of the grant of the patent: **16.05.2007**
(21) Application number: 02748386.6
(22) Date of filing: 04.03.2002
(51) Int. Cl.: A61K 38/19, A61P 1/00

(54) **STABLE AQUEOUS SOLUTIONS OF GRANULOCYTE MACROPHAGE COLONY-STIMULATING FACTOR**
STABILE WÄSSRIGE LÖSUNGEN DES GRANULOZYTEN-MAKROPHAGEN-KOLONIE-STIMULIERENDEN FAKTORS
SOLUTIONS AQUEUSES STABLES DE FACTEUR DE STIMULATION DES GRANULOCYTES ET MACROPHAGES

(30) Priority: 05.03.2001 US 800016
(43) Date of publication of application: 02.01.2004
(73) Proprietor: Bayer Schering Pharma Aktiengesellschaft, 13353 Berlin (DE)
(72) Inventor: PETTIT, Dean, K., Seattle, WA 98105 (US); JOCHHEIM, Claudia, M., Seattle, WA 98177 (US)
(74) Representative: Jones, Nicholas Andrew
(86) International application number: PCT/US2002/006428
(87) International publication number: WO 2002/069999

(56) References cited:
- EP-A2- 0 183 350
- WO-A1-92/13548
- US-A- 5 130 418
- US-A- 5 217 954
- US-A- 5 545 536
- Immunex Corp. (US) "Leukine sargramostim package insert" February 1998, pages 1-30, XP002950494
- CALLARD, R. ET AL.: 'The cytokine facts book', 1994, ACADEMIC PRESS, SAN DIEGO XP002950495 * page 139 - page 140 *

## Description

### FIELD OF THE INVENTION

The invention provides stabilized formulations of GM-CSF.

### BACKGROUND

Granulocyte macrophage colony-stimulating factor (GM-CSF) is a hematopoietic growth factor (cytokine) that stimulates the proliferation and differentiation of various hematopoietic progenitor cells in the myeloid lineage and also activates or enhances many of the functional activities of mature neutrophils, monocytes, dendritic cells and macrophages. GM-CSF is a naturally occurring glycoprotein produced, for example, by T cells, macrophages, fibroblasts and endothelial cells.

Because it affects both the supply and function of neutrophils, monocytes and dendritic cells, this cytokine plays a vital role in the body's ability to mount an immune response. GM-CSF also actes with other cytokines to promote the proliferation and differentiation of megakaryocytic and erythroid progenitors. GM-CSF activates or enhances many functional activities including chemotaxis, phagocytosis and antibody dependent cytotoxicity of mature neutrophils, monocytes, dendritic cells and macrophages.

The cDNA encoding human GM-CSF has been cloned and the recombinant protein has been produced in various expression systems including yeast, bacteria (molgramostim) and Chinese hamster ovary cells (regramostim). LEUKINE^{®} (Immunex Corporation, Seattle, Washington, U.S.A.) is a variant form of GM-CSF produced in *Saccharomyces cerevisiae*. This variant form of GM-CSF is generically termed "sargramostim."

LEUKINE^{®} has been shown to exhibit the same hematopoietic effects as those induced by endogenous GM-CSF, namely, the stimulation of progenitor cells committed along the granulocyte-macrophage pathway to form neutrophils, monocytes, macrophages, and eosinophils (Technical Product Report: LEUKINE^{®} Liquid, Immunex Corp., Seattle, WA, 1997, which is herein incorporated by reference). LEUKINE^{®}, like endogenous GM-CSF, also promotes the differentiation of progenitor cells giving rise to erythrocytes and megakaryocytes (*Ibid*.) In addition to stimulating hematopoiesis, LEUKINE^{®} enhances many of the functional activities of mature neutrophils, monocytes and macrophages, such as chemotaxis, growth factor secretion, anti-tumor activity, antibacterial and antifimgal activities, and so on (*Ibid*.).

Common uses of LEUKINE^{®} include treating recipients of autologous bone marrow transplant (non-Hodgkin's lymphoma; treating acute lymphoblastic leukemia; Hodgkin's disease); use in patients with engraftment delay or graft failure after allogeneic or autologous bone marrow transplant; treating recipients of allogeneic bone marrow transplant from an HLA-matched donor; and mobilizing peripheral blood progenitor cells in non-Hodgkin's lymphoma, Hodgkins' disease and breast cancer patients.

GM-CSF has been proposed as an effective treatment for inflammatory bowel diseases, including Crohn's disease (WO 00/47195).

Stable solutions of LEUKINE^{®} or other forms of biologically active GM-CSF are highly desired for a variety of uses.

### SUMMARY OF THE INVENTION

Provided herein are methods for long-term stabilization of GM-CSF stored in aqueous solution. Stabilization is accomplished by adding a chelating agent that is capable of forming complexes with divalent cations.

### DETAILED DESCRIPTION OF THE INVENTION

When long-term stability studies were conducted on GM-CSF that was stored in ready-to-inject syringes, it was surprisingly observed that after long periods of time the protein became partially degraded at its amino terminus (see Example 1). *In vitro* tests using a GM-CSF sensitive cell line indicated that the partially degraded preparations retained a high level of bioactivity (see Example 1). Even so, N-terminally degraded forms of GM-CSF may possess undesirable alterations in their *in vivo* pharmocologic properties. For example, it is possible that each degradation product would be metabolized somewhat differently in the patient's body. For example, the half-life *in vivo* of N-terminally degraded forms might differ from that of the full-length protein. In a therapeutic regimen, the dose and frequency of administration generally are adujsted to ensure that a therapeutically effective level of drug is maintained in the patient's body throughout the course of treatment. However, if degraded forms are present, it is possible that the needed level of drug will not be achieved and the effectiveness of the therapy could thereby be reduced. Moreover, it is theoretically possible that degraded forms of the protein could induce antibodies against GM-CSF. Accordingly, it is advantageous to have formulations of GM-CSF in which N-terminal degradation does not occur. To address this goal, studies were conducted to devise a means for stabilizing pharmaceutical preparations of GM-CSF.

This invention provides stable formulations of GM-CSF that are highly resistant to N-terminal degradation upon storage at 2-8°C. It is demonstrated herein that the addition of a chelating agent to a solution of GM-CSF prevents N-terminal degradation of the GM-CSF for periods up to 2 years. The formulations of the present invention are useful for all medical or research purposes involving GM-CSF.

If the stabilized formulations are for therapeutic administration to humans or other mammals, the chelating agent used to stabilize GM-CSF must be physiologically acceptable, that is, it must be safe for use in humans at the concentrations required for accomplishing stabilization. The chelating agent must be capable of forming complexes with divalent or trivalent cations, including ionic forms of calcium, manganese, magnesium, iron, zinc, lead, mercury, aluminum, cadmium, copper and so on. Chelating agents that are capable of chelating divalent cations are employed in one aspect of the invention. A suitable agent that chelates divalent cations is ethylenediaminetetraacetic acid, also called "EDTA" or "edetate." EDTA is used medically, for example, as a therapeutic agent to treat patients with lead poisioning. Another suitable chelating agent is dexrazoxane, which is a derivative of EDTA that readily penetrates cell membranes, and is used as a cardioprotective agent against side effects of anthracycline-induced cardiomyopathy. Additional chelating agents suitable for use in the subject formulations include dimercaprol (BAL) or BAL-glycoside derivatives, deferoxamine mesylate, deferiprone and penicillamine (dimethylcysteine). Alternatively, EGTA or citrate may be used as chelators for divalent cations in accord with the invention.

Suitable concentrations of chelating agent for the present formulations range from 0.05 to 50 mM. Suitable ranges include 0.5 to 10 mM, 0.05 to 1 mM and 0.1 to 5 mM. In one embodiment of the invention, the chelating agent is EDTA added at a concentration of 5 mM. Alternatively, a concentration of 0.1 mM EDTA may be used. Any form of EDTA may be used, for example, the dihydrate form of the disodium salt of EDTA. CaNa₂EDTA may be used.

The aqueous formulations of the invention may be packaged in vials or in ready-to-inject syringes. In one embodiment, the GM-CSF in aqueous solution is contained in a standard vial for injection. In one suitable formulation, the vials contain 1 ml of liquid comprising 500 µg/ml of GM-CSF (such as LEUKINE Liquid^{®}, Immunex Corporation), 1.2 mg/ml TRIS-HCL (also called "tromethamine"), 40 mg/ml mannitol and 10 mg/ml sucrose at pH 7.4. The foregoing formulation may also include 1.1% benzyl alcohol as a preservative, though another physiologically acceptable preservative may be substituted if desired for the benzyl alcohol. In another embodiment of the invention, the GM-CSF is packaged for storage in a ready-to-inject syringe, which contains a formulation containing GM-CSF (such as LEUKINE^{®}) at 500 µg/ml, 10 mM TRIS-HCL, 40 mg/ml mannitol and 10 mg/ml sucrose at pH 7.4. GM-CSF packaged in syringes may also include 1.1% benzyl alcohol or another pharmacologically acceptable preservative. If desired, the concentration of GM-CSF in such formulations may be increased to a concentration between 500 to 1000 µg/ml or higher.

The chelating agent is added to solutions of GM-CSF at any desired stage of preparing the formulations. For example, a concentrated solution of GM-CSF may be mixed to achieve the desired final concentration by being added to appropriate amounts of water, TRIS-HCL, mannitol, sucrose and chelating agent just prior to packaging in individual vials or syringes, or just prior to lyophilization. Standard methods of lyophilization may be used for this purpose.

GM-CSF used in the practice of the invention includes any pharmaceutically safe and effective human GM-CSF, or any derivative thereof having the biological activity of human GM-CSF. Biological activity may be ascertained, for example, by using the TF-1 cell assay described in Example 1 or other suitable bioassays known in the art. Preferably, recombinant GM-CSF is used. A suitable GM-CSF for use in the subject formulations is the human GM-CSF whose amino acid sequence is provided in SEQ ID NO:1. As used herein, the term "recombinant GM-CSF" refers to either to GM-CSF that is synthesized in a cell into which a nucleic acid encoding exogenous GM-CSF has been introduced, or a cell in which the endogenous GM-CSF gene has been stimulated to overproduce GM-CSF by the introduction of regulatory elements that induce a high rate of transcription of the endogenous GM-CSF gene.

In one embodiment of the invention, the GM-CSF used in the subject formulations is recombinant human GM-CSF (rhu GM-CSF), such as LEUKINE^{®} (Immunex Corporation, Seattle, Washington). LEUKINE^{®} (generically termed "sargramostim") is a biosynthetic, yeast-derived, recombinant human GM-CSF, consisting of a single 127 amino acid glycoprotein that differs from the endogenous human GM-CSF shown in SEQ ID NO:1 by having a leucine instead of an arginine at position 23. LEUKINE^{®} is produced in the yeast *Saccharomyces cerevisiae*. Other natural and synthetic GM-CSFs, and derivatives thereof having the biological activity of natural human GM-CSF, may be equally useful in the practice of the invention.

LEUKINE^{®} Liquid is a sterile injectable aqueous solution generally sold in 1 ml vials containing 500 µg/ml (2.8 x 10⁶ IU) sargramostim; 40 mg/ml mannitol; 10 mg/ml sucrose; 1.2 mg/ml tromethamine; sterile water; and 1.1 % benzyl alcohol. LEUKINE^{®} Lyohphilized is sold also (Immunex Corporation), and typically is packaged in vials containing a sterile lyophilized powder for reconstitution with 1 ml sterile water. LEUKINE^{®} Lyophilized may contain 250 µg or 500 µg sargramostim (1.4 or 2.8 x 10⁶ IU); 40 mg mannitol; 10 mg sucrose; and 1.2 mg tromethamine. LEUKINE^{®} Liquid and reconstituted solutions of LEUKINE^{®} Lyophilized are stored refrigerated at 2-8°C. To stabilize these formulations, a chelating agent is added as described above. For example, EDTA (or another suitable chelating agent) is added to the desired concentration to the liquid formulation before it is packaged into vials. Solid EDTA or other chelating agent may be added to the lyophilized formulation in amounts that will provide the desired final concentration when the powder is hydrated for injection.

In one aspect of the invention, provided is a process for preparing a stablized aqueous solution of granulocyte macrophage colony-stimulating factor. This process involves mixing together EDTA at a concentration of 0.1 to 50 mM, 500 µg/ml granulocyte macrophage colony-stimulating factor, 10 mM TRIS-HCL, 40 mg/ml mannitol and 10 mg/ml sucrose. The several ingredients of this mixture may be added simulteneously or sequentially in aqueous solution. The mixture may include other physiologically acceptable ingredients if so desired.

GM-CSF, such as LEUKINE^{®} or molgramostim, also can be formulated into hydrogels for topical application, such as the hydrogels described in U.S Patent No. 6,120,807. Polymers used to formulate suitable hydrogels include polysaccharides, polyacrylic acids, polyphosphazenes, polyethylene glycol-PLGA copolymers and other synthetic biodegradable polymers. To stabilize GM-CSF dispersed within such a hydrogel, a chelating agent such as EDTA is added at a concentration of 0.05 to 50 mM or at a concentration of 0.1 to 5 mM. In certain embodiments, a concentration of 0.1 or 5 mM is used.

In one embodiment of the subject invention, sargramostim is formulated as described above for LEUKINE^{®} Liquid except that EDTA is added to a concentration of 5 mM. Alternatively, sargramostim is formulated as described above for LEUKINE^{®} Lyophilized, except that 5 mM EDTA is added to the solution prior to lyophilization. In yet another embodiment, dry EDTA powder in appropriate amounts may be mixed with lyophilized LEUKINE^{®} before it is packaged.

The stable formulations of GM-CSF of the subject invention includes aqueous solutions that can be administered by any desired means. For example, the stabilized formulations of GM-CAF are administered by injection. Injection may be subcutaneous, intramuscular or by intravenous infusion.

Formulations of GM-CSF containing EDTA also may be administered by inhalation of an aerosol spray. The formulation may be packaged initially into an aerosol delivery device, or may be transferred into a container compatible with this form of delivery. Aerosol delivery may be used to dispense formulations packaged originally in liquid form or those packaged originally as a lyohphilized powder requiring reconstitution prior to being administered. Aerosol delivery is especially effective for delivering sargramostim to the nasal passages or lungs, but may be used also for systemic administration of the stabilized GM-CSF. In one embodiment of the invention, the GM-CSF administered by aerosol spray is sargramostim.

As the degree of glycosylation of biosynthetic GM-CSFs appears to influence half-life, distribution, and elimination, the most effective dose of GM-CSF for the subject methods may vary depending on the source used (Lieschke and Burgess, *N. Engl. J. Med.* **327**:28-35, 1992; Dorr, R.T., *Clin. Ther*. **15**:19-29, 1993; Horgaard et al., *Eur. J. Hematol.* **50**:32-36, 1993). The most efffective dose and frequency of administration may be adjusted as needed by the patient's physician in accord with medical practice, and will depend on the patient's age, weight and the condition being treated. Effective doses of GM-CSF may range from about 50 to 250 µg per dose. In one aspect of the invention, the dose is equal or about 100 µg. In other embodiments, the dose used is between 100 and 125 µg. In another embodiment, a flat dose ranging from 125 to 250 µg is administered. Suitable flat doses include 100 µg, 150 µg , 200 µg and 250 µg of GM-CSF. If desired, dose may be calculated as a function of body surface area, such as, for example, 125 to 250 µg/m².

The improved GM-CSF formulations described herein are useful for treating any medical condition for which the administration of GM-CSF is effective in bringing about a measurable improvement in at least one indicator that is commonly used to assess the severity of that condition. For example, the stabilized formulations of the invention can be substituted in any therapeutic regimen that utilizes LEUKINE^{®} (sargramostim), LEUCOMAX^{®} (molgramostim), regramostim, pegylated GM-CSF or any physiologically acceptable formulation of wild-type or biologically active GM-CSF. Diseases that can be treated with the stabilized GM-CSF formulations described herein include HIV infection or other viral infections, bacterial infections, cancer, slow-healing wounds or ulcers (such as decubitus ulcers or diabetic ulcers), inflammatory bowel disease, including Crohn's disease, and alveolar proteinosis. Cancers can also be treated with the subject formulations, including but not limited to melanoma, breast cancer, brain tumors, leukemias, lymphomas, carcinoma and adenocarcinoma. In addition, the formulations of the invention can be used as a vaccine adjuvant that can be administered, for example, in conjunction with a vaccine against an infectious disease, or in conjunction with a tumor vaccine, including peptide vaccines against melanoma, breast cancer, brain tumors or other cancers.

The subject stabilized GM-CSF formulations furthermore can be used for decreasing the incidence of infection in cancer patients who are receiving myelosuppressive chemotherapy; for promoting myeloid cell recovery in patients who have received myeloablative chemotherapy followed by autologous or allogeneic bone marrow transplant as treatment for cancers such as non-Hodgkin's lymphoma, acute lymphoblastic leukemia, Hodgkin's disease or other cancers; for promoting mobilization of peripheral blood progenitor cells for collection by leukapheresis prior to transplantation; for reducing the duration of neutropenia and neutropenia-related clinical sequelae in cancer patients who have received allogeneic or autologous bone marrow transplant; and for reducing the time required for neutrophil recovery in cancer patients, such as acute myelogenous leukemia patients, following chemotherapy. The stabilized formulations described herein can be used also for stimulating the extracorporeal expansion of cultured hematopoietic stem cells.

The following examples illustrate useful aspects of the invention.

### EXAMPLE 1

### N-terminal Degradation of Stored GM-CSF

Changes in the reversed-phase HPLC profile of GM-CSF were noted following about six months of storage of LEUKINE^{®} in CARPUJECT^{®} ready-to-inject syringes (AMTEST Laboratories, Redmond, WA) that had been loaded with 1 ml of LEUKINE^{®} Liquid. Reversed-phase HPLC for these analyses was performed using a Vydac Protein and Peptide C18(#218TP54) column, 5 µm, 4.6 x 250 mm. Buffer A for the reversed-phase chromotography was water/TFA 0.1%; buffer B was acetonitrile/TFA 0.1%; and buffer C was 1M NaCl/water/TFA 0.1%. The gradient used to develop the column was: 1% B/min for 25-65%, B at a constant 20% and C for 40 minutes at 1 ml/min. Injection volume was 50 µl. The elution profile for the stored LEUKINE^{®} showed a descending shoulder on the major peak of this profile.

Mass spectrometry identified this shoulder as GM-CSF that had been clipped to varying degrees at the N-teminus. Mass spectrometry (Sciex API 350) was performed by developing samples on C18 reversed-phase columns as described above except that sodium chloride was omitted from the buffers. Eluate from the C18 column was electrosprayed into the mass spectrometer. For analyzing mass spectra, mass/charge (m/z) spectra were taken off the entire eluted GM-CSF peak and deconvoluted to masses by using BioMultiView software.

LEUKINE^{®} in its unmodified formulation is heterogeneous at its amino terminus, typically comprising 65% full length protein, and 35% of a slightly smaller form beginning at Ala3. Mass spectra results for the syringe-stored products indicated the presence of the mature Ala1 species as well as additional species clipped to Ala3, Arg4, Ser5, Ser7, Ser9 and Thr10. In products stored at 2-8°C, the proportion of the full-length protein (Ala1) remained unchanged, indicating that the only species susceptible to storage-induced degradation at this temperature is the Ala3 species. However, at 30°C, even the Ala1 species became degraded. It should be noted that these mass spectra analyses were focused on only the non-glycosylated species, though later work showed that the glycosylated forms are also clipped. The limit of quantitation in determining the percentage of full length and clipped species by this method (LOQ) is estimated to be about 5%.

Further characterizations suggested that this clipping was due to a metal ion catalyzed process. Supporting this conclusion were the following observations. First, degradation was isolated to the N-terminus. Second, the reverse-phase shoulder could be simulated by the addition of an exogenous α-aminopeptidase. For this simulation, 5 units of α-aminopeptidase (Sigma A8299) were added to LEUKINE^{®} Liquid, and this was incubated for three days at 37°C. Formulations with added α-aminopeptidase upon mass spectrometry were found to contain the following N-termini: Ser5, Gln11 and pGln11 (the stable cyclization product of Gln11). When the incubation was extended to as long as seven days, degradation did not proceed beyond Gln11. Further degradation may have been arrested by the cyclization of Gln11. Third, we were able to arrest the observed N-terminal degradation of GM-CSF by adding mM EDTA to the formulations.

The cation that catalyzes this amino-terminal degradation has not been definitively identified. In an effort to identify this cation, metal ions were exhaustively extracted from rubber stoppers taken from CARPUJECT^{®} syringes by using soxhelet extraction. The most abundant divalent cation that was extracted was zinc, thus suggesting that this might be the catalytic cation. Experiments were conducted in which Zn²⁺ was added to formulations of LEUKINE^{®} in an effort to accelerate the N-terminal degradation, but the added zinc had no significant effect as compared with a control sample. However, these samples were stored for only about a month prior to analysis, so the results were not considered to be conclusive. According to information provided by the manufacturer of CARPUJECT^{®} syringes, many different metal ions are present in small amounts in the rubber stoppers, including calcium, copper, iron, lead, chromium, magnesium, manganese, molybdenum and others.

Bioassays were performed using the cell line TF-1, which is sensitive to human GM-CSF and proliferates more rapidly when this cytokine is added to the culture medium (see, for example, Kitamura et al., *J Cell Physiol* 140:323-334 (1989)). Bioactivity is assayed by adding known quantities of GM-CSF (1 ng/ml) to the cells in the presence of H³-thymidine. Cell proliferation in response to the GM-CSF is quantified by measuring the amount of tritiated thymidine incorporated into DNA by the cells. Results of bioassays on the clipped forms of sargramostim indicated that when it was clipped to intermediate positions (combinations of Arg4, Ser5, Ser7, Ser9 and Thr10) as well when it was clipped completely to Gln11, no significant change in biological activity was observed.

### EXAMPLE 2

### Long-term Stability Testing

To test the long-term effects of storage in the presence of EDTA, a total of seven lots of LEUKINE^{®} were set up in CARPUJECT^{®} syringes with and without the addition of 5 mM EDTA. The syringes were stored at either 2-8°C (normal storage temperature) or 30°C (to induce accelerated degradation). Each syringe contained 1 ml of liquid containing 500 µg of LEUKINE^{®} and 10 mM TRIS-HCL (1.2 mg/ml), 40 mg/ml mannitol, and 10 mg/ml sucrose at pH 7.4. After incubation for varying lengths of time, stored samples were analyzed for N-terminal degradation.

At six months, samples were analyzed by SDS-PAGE, reversed-phase HPLC, TF-1 bioassay, reduced and non-reduced tryptic peptide mapping and mass spectrometry (Sciex API 350). For SDS-PAGE, sample loads were 1 µg/lane in 2X phosphate non-reducing sample buffer on Novex 16% TRIS-glycine gels. Gels were run at 30 mA in TRIS-glycine SDS running buffer and stained using Novex silver Xpress staining kit. For reversed-phase HPLC, we used a Vydac Protein and Peptide C18(#218TP54) column, 5 µm, 4.6 x 250 mm. Buffer A was: Water/TFA 0.1%, and buffer B was: acetonitrile/TFA 0.1%, buffer C: 1M NaCl/water/TFA 0.1%. The gradient was: 1% B/min for 25-65%, B at a constant 20%, and C for 40 minutes at 1 mL/min. Injection volume was 50 µl. The TF-1 assays were performed as described for Example 1. For mass spectrometry, samples were developed on C18 reversed-phase columns developed as above but without sodium chloride, then electrosprayed into the mass spectrometer. Mass spectra results were used to provide semi-quantitative data on the degree of N-terminal degradation, though this method was not validated. The limit of quantitation in determining the percentage of full length and clipped species by this method is estimated to be about 5%. Reduced tryptic peptide mapping identifies C-terminal peptides that are disulfide linked. The peptide mapping was done by reversed phase-analysis on C18 of trypsinized protein.

Results of the six month analyses demonstrated a clear N-terminal degradation when LEUKINE^{®} was stored in syringes at either 2-8 °C or 30 °C for six months in the absence of EDTA. The extent of degradation varied considerably from lot to lot. For samples incubated at 2-8 °C in the presence of either 0.1 or 5 mM EDTA, N-terminal degradation was eliminated. For samples incubated at 30 °C in the presence of either 0.1 or 5 mM EDTA, N-terminal degradation was significantly reduced. It was also noted that for a single lot of LEUKINE^{®}, samples incubated at 30 °C in the presence of high concentration EDTA (5 mM) demonstrated an unexplained mass loss of approximately 17 Da, but the did not occur in any of the other six lots that were tested.

At 12 months, samples from seven lots stored with or without EDTA at 2-8°C or with or without 5 mM EDTA at 30°C were analyzed. This batch of samples also included one lot that had been formulated with 0.1 mM EDTA. These samples were analyzed by SDS-PAGE (4-20% Novex gels using non-reducing sample buffer and run at 34 mA), reversed-phase HPLC run as for the six month samples, TF-1 bioassay (see below) and mass spectrometry. The results indicated that compared with the six month results, N-terminal degradation had continued at both temperatures in samples stored for 12 months without EDTA. Formulations containing EDTA at either concentration (0.1 or 5 mM) were protected from degradation when stored at 2-8°C. N-terminal degradation did occur in 12 month samples stored at 30°C with or without added EDTA.

One of the test lots was analyzed at 1, 3, 6 and 12 months. In this particular lot, samples stored at 2-8°C without EDTA exhibited a time-dependent course of degradation, and the Ala3 species was entirely gone by 12 months. In one of the seven lots tested, no loss of the Ala3 species was observed in the absence of EDTA, though the reason for this is not known. In brief, six of the seven tested lots did exhibit N-terminal degradation when stored in syringes at 2-8°C in the absence of EDTA.

For the 24 month time point, four lots stored with or without EDTA at 2-8°C and four lots stored with EDTA at 30°C were analyzed. These samples were analyzed by SDS-PAGE, RP-HPLC, SEC, TF-1 bioassay, and mass spectrometry as described above in Example 1 or for the six month samples. For the samples stored for 24 months at 2-8°C, complete N-terminal degradation of the Ala3 species was observed in the absence of EDTA. Samples stored at 2-8°C in the presence of 5 mM EDTA did not exhibit this degradation. Degradation of the full-length (Ala1) species was seen when the samples were stored at 30°C with EDTA. It is concluded that LEUKINE® formulations containing 5 mM EDTA can remain stable for 2 years when stored at 2-8°C.

### EXAMPLE 3

### Effect of EDTA Concentration in Stabilizing GM-CSF

Leukine was stored at 2-8°C or 30°C for 14 months in CARPUJECT® syringes with 0, 0.1, 1.5, 5, 10, or 50 mM EDTA.

After six weeks, some of the samples were analyzed by non-reduced SDS-PAGE, reversed-phase HPLC, mass spectrometryand reduced and non-reduced peptide mapping as described in the previous examples. After 14 months, the remaining samples were analyzed by SDS-PAGE, size-exclusion chromatography (SEC) on BIORAD^{®} Biosil columns, reversed-phase HPLC using a Vydac Protein and Peptide C18 column, TF-1 bioassay, and mass spectrometry as described above, except that for size exclusion chromatography (SEC), 20 µl of each sample was injected into a Biorad Biosil 125 column and eluted isocratically using 100 mM sodium phosphate, 150 mM NaCl, pH 6.8 at 1 ml/min as the mobile phase. The 14 month analyses included samples stored at 2-8°C with and without EDTA, and stored at 30°C without EDTA.

The results confirmed that EDTA acts to preserve LEUKINE^{®} against N-terminal clipping in samples stored at 2-8°C for 14 months. At this time point, samples stored at 30°C with 10 mM EDTA was no longer available for analysis, but all the other samples were analyzed by all of the above-described methods except for peptide mapping analysis. Samples stored at 30°C showed a decrease in the proportion of full-length (Ala1) species compared to the samples stored at 2-8°C, and the N-terminal degradation was worst in the sample stored without EDTA at 30°C. Samples stored at 30°C also showed extensive oxidation, evidenced by a large portion of early-eluting material seen by reversed-phase HPLC. For samples stored at 2-8°C, concentrations of EDTA as low as 0.1 mM were effective at inhibiting the N-terminal degradation of GM-CSF.

Table 1 below presents estimates based on mass spectrometry after 14 months of storage. The numbers in Table 1 indicate the percentage of the total protein analyzed that was represented by each of the species listed in the table. Without EDTA, 24% of the GM-CSF stored at 2-8°C was shortened to Arg4 or Ser5. In all of the samples stored with EDTA at 2-8°C, regardless of the EDTA concentration, no species smaller than Arg4 were observed, and only 5% or less of the Leukine ended in Arg4. The samples stored in the presence of EDTA at 2-8°C consisted of 57-71% full-length (Ala1) GM-CSF. Since 0.1 mM EDTA was as protective as the higher concentrations tested, it is possible that concentrations of EDTA lower than 0.1 mM would also be effective in preventing the N-terminal degradation.

In all samples stored at 30°C there was a great amount of N-terminal clipping whether or not EDTA was present (see Table 1). For example, in the sample stored without EDTA at 30°C, 61% of the protein was clipped to Arg4 or further. Clipped species out to Trp13 were found. In contrast, the samples stored at this temperature with EDTA showed only 20-30% of species clipped to Arg4 or beyond, with no clips beyond Thr10. The degradation included a substantial reduction in the full-length (Ala1) species, with only 28-39% of the full length species remaining after 14 months at 30°C.

Samples were analyzed in the TF-1 bioassay data to assess bioactivity compared to a reference sample of GM-CSF. The sample stored without EDTA at 30°C showed anomalously high activity. Results indicated that there may have been a slight decrease in bioactivity in the samples stored at 30°C in the presence of EDTA, but in any case there was not a clear trend showing that bioactivity increased in this group of samples with increasing amounts of EDTA. All samples stored at 2-8°C with or without EDTA showed bioactivity comparable to the reference sample of LEUKINE^{®}.

The above studies confirmed that EDTA at concentrations ranging from 0.1 to 50 mM acts to protect GM-CSF against N-terminal clipping in samples stored at 2-8°C for up to 14 months. Samples stored at 30°C showed a decrease in the proportion of full-length (Ala1) species compared to the samples stored at 2-8°C, and the N-terminal degradation was most extreme in the sample stored without EDTA at 30°C. Samples stored at 30°C also showed extensive oxidation, as evidenced by a large portion of early-eluting material seen by reversed-phase HPLC.

**Table 1. Mass Spectrometry Analyses of LEUKINE^{®} stored 14 months at 2-8°C or 30°C**

| | **0 mM EDTA** | **0.1 mM EDTA** | **1.5 mM EDTA** | **5 mM EDTA** | **10 mM EDTA** | **50 mM EDTA** |
|---|---|---|---|---|---|---|
| **2-8°C** | | | | | | |
| % A1 | 57 | 65 | 63 | 71 | 62 | 62 |
| % A3 | 19 | 35 | 32 | 29 | 34 | 33 |
| % R4 | 15 | 0 | 5 | 0 | 4 | 5 |
| % S5 | 9 | 0 | 0 | 0 | 0 | 0 |
| % S7 | 0 | 0 | 0 | 0 | 0 | 0 |
| % S9 | 0 | 0 | 0 | 0 | 0 | 0 |
| % T10 | 0 | 0 | 0 | 0 | 0 | 0 |
| % P12 | 0 | 0 | 0 | 0 | 0 | 0 |

| | **0 mM EDTA** | **0.1 mM EDTA** | **1.5 mM EDTA** | **5 mM EDTA** | **10 mM EDTA** | **50 mM EDTA** |
|---|---|---|---|---|---|---|
| **30°C** | | | | | | |
| % A1 | 28 | 35 | 39 | 32 | - | 34 |
| % A3 | 11 | 43 | 39 | 43 | - | 35 |
| % R4 | 13 | 9 | 0 | 0 | - | 0 |
| % S5 | 11 | 7 | 6 | 8 | - | 11 |
| % S7 | 13 | 6 | 6 | 6 | - | 10 |
| % S9 | 8 | 0 | 6 | 6 | - | 9 |
| % T10 | 8 | 0 | 4 | 5 | - | 0 |
| % P12 | 4 | 0 | 0 | 0 | - | 0 |
| % W13 | 4 | 0 | 0 | 0 | - | 0 |

| | | | | | | |
|---|---|---|---|---|---|---|
| - : no sample | | | | | | |

While exemplary embodiments of the invention have been illustrated and described above, it will be appreciated that various changes can be made therein without departing from the spirit and scope of the invention.

### SEQUENCE LISTING

<110> IMMUNEX CORPORATION
   PETTIT, Dean, K.
   JOCHHEIM, Claudia, M.
<120> STABLE AQUEOUS SOLUTIONS OF GRANULOCYTE MACROPHAGE COLONY-STIMULATING FACTOR
<130> 3253-WO
<140> --to be assigned--
   <141> 2002-03-04
<150> 09/800,016
   <151> 2001-03-05
<160> 1
<170> PatentIn version 3.1
<210> 1
   <211> 127
   <212> PRT
   <213> Homo sapiens
<400> 1

## Claims

1. A physiologically acceptable aqueous solution that comprises recombinant granulocyte macrophage colony-stimulating factor and from 0.1 mM to 50 mM EDTA.

2. The aqueous solution of claim 1, wherein the concentration of EDTA is 0.1 to 5 mM.

3. The aqueous solution of claim 1, wherein the recombinant granulocyte macrophage colony-stimulating factor is sargramostim.

4. The aqueous solution of any one of claims 1 to 3, wherein the EDTA concentration is 5 mM, and further wherein the solution has a pH of 7.4 and comprises 10 mM TRIS-HCL, 40 mg/ml mannitol and 10 mg/ml sucrose.

5. The aqueous solution of any one of claims 1 to 3 comprising GM-CSF at 500 µg/ml, 10 mM TRIS-HCL, 40 mg/ml mannitol, 10 mg/ml sucrose at pH 7.4, and 1.1% benzyl alcohol.

6. A process for preparing a stabilized, physiologically acceptable, aqueous solution of granulocyte macrophage colony-stimulating factor, which comprises mixing EDTA to a concentration of 0.1 to 50 mM with an aqueous solution comprising 500 µg/ml granulocyte macrophage colony-stimulating factor, 10 mM TRIS-HCL, 40 mg/ml mannitol and 10 mg/ml sucrose.

7. A process for preparing a lyophilized formulation of recombinant granulocyte colony-stimulating factor, which comprises:
a) preparing the aqueous solution of any one of claims 1 to 5; and
b) lyophilizing the aqueous solution.

8. A process for preparing a lyophilized formulation of recombinant GM-CSF, comprising:
a) lyophilizing an aqueous solution according to any one of claims 1 to 5, but without EDTA; and
b) adding an appropriate amount of EDTA to form a lyophilized formulation that when hydrated, produces the aqueous solution according to any one of claims 1 to 5.

9. The process of any of claims 6 to 8, wherein the recombinant granulocyte macrophage colony-stimulating factor is sargramostim.

10. The aqueous solution of any one of claims 1 to 5, for use in therapy.

11. Use of the aqueous solution of any one of claims 1 to 5 in the preparation of a medicament for treating inflammatory bowel disease.

12. Use of claim 11 wherein the inflammatory bowel disease is Crohn's disease.

13. Use of the aqueous solution of any one of claims 1 to 5 in the preparation of a medicament for treating ulcers.

14. A lyophilized formulation of recombinant granulocyte macrophage colony-stimulating factor obtainable according to the process of claim 7 or claim 8.

## Patentansprüche

1. Physiologisch verträgliche wässrige Lösung, die rekombinanten Granulozyten-Makrophagen-Kolonie-stimulierenden Faktor und von 0,1 mM bis 50 mM EDTA umfasst.

2. Wässrige Lösung nach Anspruch 1, wobei die Konzentration an EDTA von 0,1 bis 5 mM beträgt.

3. Wässrige Lösung nach Anspruch 1, wobei der rekombinante Granulozyten-Makrophagen-Kolonie-stimulierende Faktor Sargramostim ist.

4. Wässrige Lösung nach einem der Ansprüche 1 bis 3, wobei die EDTA-Konzentration 5 mM ist, und weiter, wobei die Lösung einen pH-Wert von 7,4 hat und 10 mM TRIS-HCl, 40 mg/ml Mannitol und 10 mg/ml Saccharose umfasst.

5. Wässrige Lösung nach einem der Ansprüche 1 bis 3, umfassend GM-CSF zu 500 µg/ml, 10 mM TRIS-HCl, 40 mg/ml Mannitol, 10 mg/ml Saccharose bei pH-Wert 7,4 und 1,1 % Benzylalkohol.

6. Verfahren zur Herstellung einer stabilisierten, physiologisch verträglichen, wässrigen Lösung des Granulozyten-Makrophagen-Kolonie-stimulierenden Faktors, umfassend Mischen von EDTA mit einer wässrigen 500 µg/ml Granulozyten-Makrophagen-Kolonie-stimulierenden Faktor umfassenden Lösung, 10 mM TRIS-HCl, 40 mg/ml Mannitol und 10 mg/ml Saccharose, auf eine Konzentration von 0,1 bis 50 mM.

7. Verfahren zur Herstellung einer lyophilisierten Formulierung von rekombinantem Granulozyten-Makrophagen-Kolonie-stimulierenden Faktor, welches umfasst:
a) Herstellen der wässrigen Lösung nach einem der Ansprüche 1 bis 5 und
b) Lyophilisieren der wässrigen Lösung.

8. Verfahren zur Herstellung einer lyophilisierten Formulierung von rekombinantem GM-CSF, umfassend:
a) Lyophilisieren einer wässrigen Lösung nach einem der Ansprüche 1 bis 5, jedoch ohne EDTA, und
b) Zusetzen einer geeigneten Menge an EDTA zur Bildung einer lyophilisierten Formulierung, die bei Hydratisierung die wässrige Lösung nach einem der Ansprüche 1 bis 5 ergibt.

9. Verfahren nach einem der Ansprüche 6 bis 8, wobei der rekombinante Granulozyten-Makrophagen-Kolonie-stimulierende Faktor Sargramostim ist.

10. Wässrige Lösung nach einem der Ansprüche 1 bis 5 zur Verwendung bei der Therapie.

11. Verwendung der wässrigen Lösung nach einem der Ansprüche 1 bis 5 bei der Herstellung eines Medikaments zur Behandlung einer entzündlichen Darmerkrankung.

12. Verwendung nach Anspruch 11, wobei die entzündliche Darmerkrankung Morbus Crohn ist.

13. Verwendung der wässrigen Lösung nach einem der Ansprüche 1 bis 5 bei der Herstellung eines Medikaments zur Behandlung von Geschwüren.

14. Lyophilisierte Formulierung von rekombinantem Granulozyten-Makrophagen-Kolonie-stimulierenden Faktor, erhältlich gemäß dem Verfahren nach Anspruch 7 oder Anspruch 8.

## Revendications

1. Solution aqueuse physiologiquement acceptable qui comprend un facteur de stimulation de colonie de macrophages granulocytaires recombinant et 0,1 mM à 50 mM d'acide éthylène diamino-tétra-acétique sodique (EDTA).

2. Solution aqueuse selon la revendication 1, dans laquelle la concentration d'acide EDTA est de 0,1 à 5 mM.

3. Solution aqueuse selon la revendication 1, dans laquelle le facteur de stimulation de colonie de macrophages granulocytaires (6M-CSF) recombinant est la sargramostime.

4. Solution aqueuse selon l'une quelconque des revendications 1 à 3, dans laquelle la concentration d'acide EDTA est de 5 mM et où en outre, la solution a un pH de 7,4 et comprend 10 mM d'acide chlorhydrique (TRIS-HCL), 40 mg/ml de mannitol et 10 mg/ml de saccharose.

5. Solution aqueuse selon l'une quelconque des revendications 1 à 3 comprenant le facteur GM-CSF à 500 µg/ml, 10 mM de TRIS-HCL, 40 mg/ml de mannitol, 10 mg/ml de saccharose à un pH de 7,4 et de l'alcool benzylique à 1,1 %.

6. Procédé de préparation d'une solution aqueuse physiologiquement acceptable stabilisée d'un facteur de stimulation de colonie de macrophages granulocytaires, qui comprend le mélange d'acide EDTA à une concentration de 0,1 à 50 mM avec une solution aqueuse comprenant 500 µg/ml de facteur de stimulation de colonie de macrophages granulocytaires, 10 mM de TRIS-HCL, 40 mg/ml de mannitol et 10 mg/ml de saccharose.

7. Procédé de préparation d'une formulation lyophilisée d'un facteur de stimulation de colonie de macrophages granulocytaires recombinant qui comprend :
a) la préparation de la solution aqueuse selon l'une quelconque des revendications 1 à 5, et
b) la lyophilisation de la solution aqueuse.

8. Procédé de préparation d'une formulation lyophilisée du facteur GM-CSF recombinant, comprenant :
a) la lyophilisation d'une solution aqueuse selon l'une quelconque des revendications 1 à 5, mais sans acide EDTA, et
b) l'ajout d'une quantité appropriée d'acide EDTA afin de former une formulation lyophilisée qui, lorsqu'elle est hydratée, produit la solution aqueuse selon l'une quelconque des revendications 1 à 5.

9. Procédé selon l'une quelconque des revendications 6 à 8, dans lequel le facteur de stimulation de colonie de macrophages granulocytaires recombinant est la sargramostime.

10. Solution aqueuse selon l'une quelconque des revendications 1 à 5, destinée à une utilisation dans une thérapie.

11. Utilisation de la solution aqueuse selon l'une quelconque des revendications 1 à 5, dans la préparation d'un médicament destiné au traitement des maladies inflammatoires chroniques de l'intestin.

12. Utilisation selon la revendication 11, dans laquelle la maladie inflammatoire chronique de l'intestin est la maladie de Crohn.

13. Utilisation de la solution aqueuse selon l'une quelconque des revendications 1 à 5, dans la préparation d'un médicament destiné à traiter des ulcères.

14. Formulation lyophilisée du facteur de stimulation de colonie de macrophages granulocytaires recombinant pouvant être obtenue selon le procédé de la revendication 7 ou de la revendication 8.
